Europäisches Patentamt

(19) European Patent Office

Office européen des brevets

(11) Publication number: **0 114 499**
**A2**

(12) # EUROPEAN PATENT APPLICATION

(21) Application number: **83307794.4**

(22) Date of filing: **21.12.83**

(51) Int. Cl.³: **A 61 B 3/16**
**A 61 B 5/04**

(30) Priority: **27.12.82 US 453605**

(43) Date of publication of application:
**01.08.84 Bulletin 84/31**

(84) Designated Contracting States:
**AT BE CH DE FR GB IT LI NL SE**

(71) Applicant: **UNIVERSITY OF NEW HAMPSHIRE**
**Main Street**
**Durham New Hamsphire 03824(US)**

(72) Inventor: **LaCourse, John R.**
**RFD No. 1 Stepping Stone Road**
**Durham New hampshire(US)**

(74) Representative: **Rushton, Ronald et al,**
**SOMMERVILLE & RUSHTON 11 Holywell Hill**
**St. Albans Hertfordshire AL1 3EZ(GB)**

(54) **A system to determine arterial occlusion and other maladies.**

(57) A system for noninvasively sensing ocular pulses of a mammal, which pulses serve as a basis for indicating presence of a disease or malfunctioning body part such as, for example, a condition of arterial occlusion. The system employs piezoelectric transducers to sense the ocular pulses which are analyzed, also, in the case of arterial occlusion with simultaneously obtained ECG signals.

FIG.6

EP 0 114 499 A2

# A SYSTEM TO DETERMINE ARTERIAL OCCLUSION
## AND OTHER MALADIES

The present invention relates to systems and methods to determine arterial occlusion and glaucoma.

By way of general background, attention is called to "Joint Study of Extracranial Arterial Occlusion. II. Arteriography Techniques, Sites and Complications," J.A.M.A. 203:961, Haas et al., (1968) and "Measurement of Internal Carotid Artery Blood Volume Pulsations by Noncontact Ocular Photoelectric Plethysmography." IEEE TRANS. Biomed. Eng., BME-28(8) 573, Rasmussen et al. (1981).

Stroke accounts for the death or disability of nearly one-half million people each year in this country and it is estimated that as many as 30 percent of these strokes are the result of stenosis or occlusion of the extracranial arteries. If this narrowing in these arteries can be detected before any permanent brain damage occurs, it usually can be surgically corrected.

Currently carotid angiography is usually employed to investigate stenosis or occlusion in the extracranial arteries. Carotid angiography is an invesive procedure wherein a catheter is inserted into the artery in situ. Although the carotid angiography procedure is the best way of demonstrating the site of an occlusive lesion, it does incur appreciable morbidity and mortality. Consequently, angiography is often not applicable for the early detection and periodic evaluation of many patients with suspected carotid occlusive disease. Therefore, there is a need for a safe, simple, and reliable diagnostic screening procedure for the noninvasive evaluation of carotid occlusive disease. During the past few years there have been many noninvasive screening tests developed for carotid occlusive disease, but no single procedure has yet emerged as preeminent. The present disclosure discribes a new system to measure the ocular pulse (OP) in humans and in other mammals and to diagnose carotid insufficiency and, in some instances, glaucoma therefrom.

The OP is the minute, radial displacement of the corneal surface of the eye caused by arterial pressure pulsations in the ocular circulation acting on the compliance of the cornea. Features of the OP waveform (i.e. shape, amplitude, duration) have been shown by several workers (Best and Rogers, "Techniques of Ocular Pulse Analysis in Carotid Stenosis," Arch. Opthalmol. 92:54 (1974); Horven and Nornes, "Crest Time Evaluation of Corneal Indentation Pulse," Arch. Opthalmol. 86:5 (1971)) to be important diagnostic indicators of cerebrovascular disease (CVD) in which the common carotid and/or lower internal carotid arteries are significantly occluded. To be more exact, with each heartbeat blood vessels supplying the eye cause a pressure wave or pulse to propagate to the cornea, producing a slight distension (1 to 50 micrometers).

Alterations in the ocular blood supply are reflected in parameters defining the ocular pulse. Differences in ocular pulses in both eyes are associated with pathology (Best et al., "Graphic Analysis of the Ocular Pulse in Carotid Stenosis," Arch. Opthalmol. 85:315 (1971); Bynke, H., "Screening Diagnosis of Carotid Occlusion by Means of Oculosphygmography," Neurology 16:383 (1966); Sand et al., "Ophthalmic Arterial Blood Pressures Measured by Ocular Pneumoplethysmography," Arch. Surg. 110:813 (1975); and Galin et al., "The Ocular Pulse," Trans. Am. Acad. Ophth. & Otol. 76:1535 (1972)). By the monitoring of the ocular pulse, that class of vascular occlusive diseases causing alterations in ocular the pulse become amenable to rapid early detection.

Accordingly, it is a principal object of the present invention to provide a simple and reliable diagnostic screening system for noninvasive evaluation of carotid occlusive disease.

Another object is to provide a system suitable to sense ocular pulses (OP) and operable to relate those pulses to a condition of carotid occlusive disease.

Still another object is to provide a system of more general use.

A secondary object of the present invention is to provide a simple and reliable diagnostic screening system for noninvasive evaluation of glaucoma.

Another object is to provide a simple and reliable diagnostic screening system for noninvasive evaluation of choroidal melanoma, carotid obstruction, giant cell arteritis, and carotid cavernous sinus fistula.

These and still further objects are addressed hereinafter.

According to the invention, there is provided apparatus for noninvasively sensing ocular pulses of a mammal, which pulses serve as a basis for indicating a condition of the mammal which causes alterations in the arterial pulses, characterized by:

piezoelectric transducer means to receive arterial pulses from the mammal and operable to convert the arterial pulses to a signal in the form of similar electric signals; and means connected to receive said signal and operable to permit deriviation of the information from which the condition of the mammal can be inferred.

The invention also includes a noninvasive method of measuring the ocular pulses in mammals, which pulses serve as a basis for determination of any stenosis or occlusion in extracranial arteries of the mammal, characterized by:

(a)　immobilizing the head of the mammal,

(b)　attaching an ECG lead to at least three limbs of the mammal, to record heartbeats of the mammal in the form of electric signals,

(c)　fitting a first piezoelectric transducer onto the surface of one eye of the mammal to convert the ocular pulse received by the first piezoelectric transducer to a first signal of similar electric pulses,

(d)　fitting a second piezoelectric transducer onto the surface of the other eye of the mammal to convert the ocular pulse received by the second piezoelectric transducer to a second signal of similar electric pulses,

(e)　displaying waveshapes of the electric signal from the ECG transducer, the first signal of similar electric pulses from the first piezoelectric transducer, and the second signal of similar electric pulses from the second piezoelectric transducer, and

(f)　comparing the series of waveshapes to permit simultaneous observation and simultaneous comparison of characteristics of the waveshapes derived from each eye and the ECG to permit an inference of any stenosis or occlusion of the extracranial arteries of the mammal being examined therefor;

The invention is hereinafter described, by way of example, with reference to the accompanying drawings, in which:

Fig. 1 is an enlarged (∿ two to one) isometric view showing mechanical portions, including a piezoelectric bender, of a part of a system according to the present teachings and includes conductors for connection to electrical circuitry thereof;

Fig. 2 is a sectional elevation view of a portion of the structure in Fig. 1;

Fig. 3 is a side view of most of the mechanical portion in Fig. 1;

Fig. 4 shows, greatly enlarged, a side view of a portion of the piezoelectric bender of Figs. 1 and 3;

Fig. 5 shows diagrammatically a system that includes both mechanical and electrical elements of a system employing the present concepts; and

Fig. 6 shows diagrammatically a system of the type shown in Fig. 5 in conjunction with a patient.

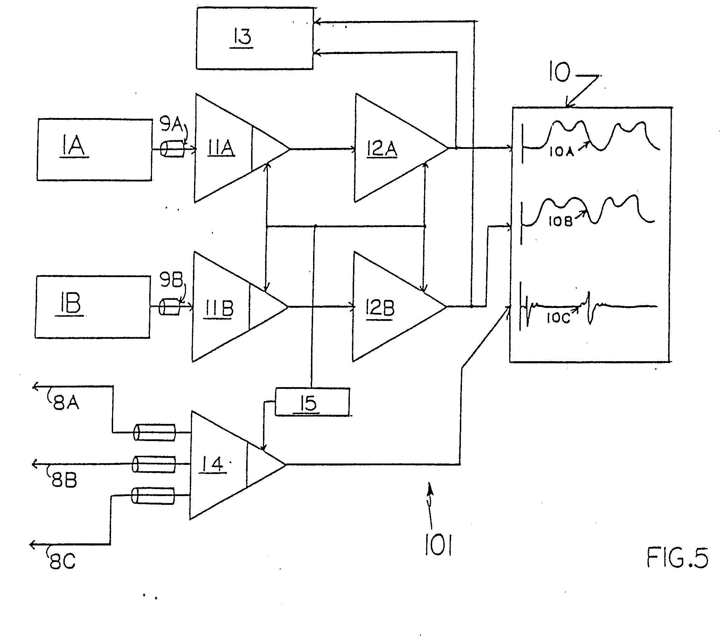

Turning now to the drawings, the mechanism (or system) labeled 101 in Fig. 5, called the Piezoelectric Ocular Pulse Measurement System (POPMS) by the present inventor, serves as a basis for determination of an occlusion of extracranial arteries of a mammal. The POPMS diagnostic mechanism, as is discussed in detail hereinafter, permits quick, noninvasive evaluation of the physical condition of the carotid artery. While emphasis herein is use of the general concepts in connection discovery of occlusion of extracranial arteries of the mammal, it is noted that the invention is broader in scope.

By way of brief introduction, the diagnostic system 101 senses ocular pulses of both the left eye and the right eye of the mammal while, at the same time recording heartbeats (i.e. the electrical activity of the heart, ECG) of the mammal. All three pulses are converted to the electric signals which are simultaneously displayed and/or stored by a device 10 which may be, for example, a strip chart recorder, an oscilloscope or a magnetic tape recorder or a combination thereof. The labels 10A, 10B and 10C in Fig. 5 designate respectively a series of waveshapes of a first signal of similar electric pulses derived from the left eye of the mammal, a second signal of similar electric pulses derived from the right eye of the mammal and ECG electric signals recording the heartbeat of the mammal.

The waveshapes of the signals 10A, 10B and 10C serve as a basis for analysis according to the present teaching. For example, a trained operator viewing the waveshpaes 10A, 10B and 10C can compare the amplitude of the waveshape derived from the left eye with the amplitude of the waveshape derived from the right eye, the areas under the waveshapes, and the delay of each waveshape from each eye with respect to the corresponding ECG signal to permit an inference of any stenosis or occlusion of the extracranial arteries of the mammal being checked therefor.

It has been shown by Galin et al (1972) and Best et al (1971), and others that the degree of reduction in the ocular pulse amplitude is related directly to the amount of carotid stenosis. Also, it has been shown by the above scientists that acute common carotid litigation caused a marked reduction in the amplitude of the homolateral ocular pulse, disappearance of the dicrotic notch, delay in crest time and reduction in the steepness of the anacrotic and catacrotic slopes. In other words, the degree of variation in the amplitude, shape and/or area under the waveform between left and right ocular pulse waveforms denote the extent of the stenosis. Walden et al, "Complementary Methods for Evaluating Carotid Stenosis: A Biophysical Basis for Ocular Pulse Wave Delays," Surgery 88(1):162 (1980), has demonstrated that the degree of delay from the ECG signal to the ocular pulse waveform indicates the degree of compliance distal to the stenosis, i.e. the physical condition of the artery. In all comparison of the characteristics of the left to right ocular pulse waveforms and comparison of both waveforms to an ECG signal, provides information to the degree of stenosis and to the physical condition of the occluded artery.

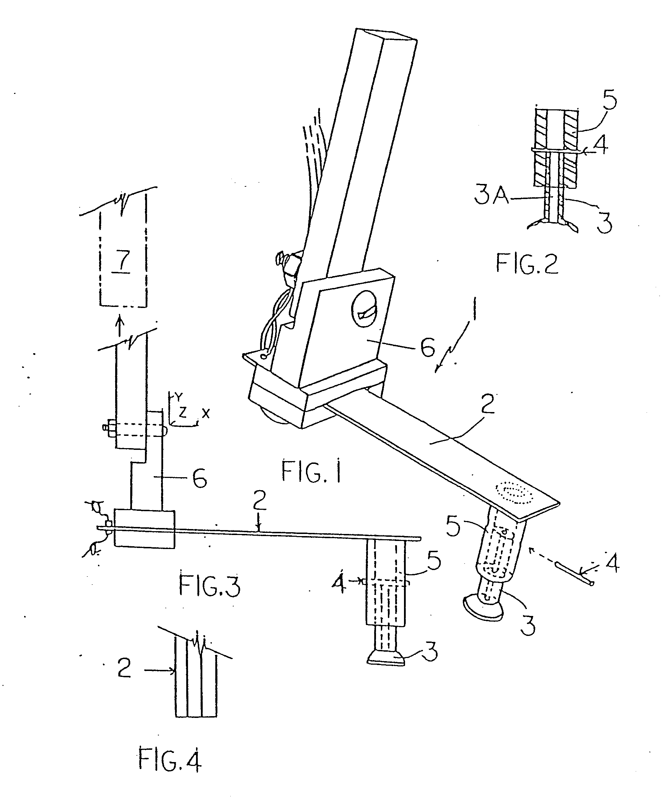

The system 101 includes a first transducer assembly 1A to receive ocular pulses from the left eye of the mammal and operable to convert the ocular pulses to a first signal of similar electric pulses (e.g., the signal represented by the trace 10A); a second transducer assembly 1B to receive ocular pulses from the right eye and operable to convert the ocular pulses to a second signal of similar electric pulses (e.g. the signal represented by the trace 10B); and ECG leads 8A, 8B and 8C to record heartbeats of the mammal as represented by the trace 10C. A most important aspect of the present invention is the two devices in the assemblies 1A and 1B, which are piezoelectric devices of the type shown at 1 in Fig. 1 and now discussed.

The device 1 uses a piezoelectric bender or transducer 2 which effects physical contact with the cornea of the eye by way of a presterilized disposable contact lens retriever 3 that has an axial hole 3A in Fig. 2 to prevent a vacuum. The peizoelectric bender 2 is a piezoceramic crystalline bender which is a low power electromechanical transducer capable of converting mechanical energy to electrical energy. The lens retriever 3 is replacably held within a sleeve 5, stopped by a pin 4 which permits facile replacement of the retriever 3 and maintains calibration after use. Ocular pulses from the eye are thereby transmitted to the piezoceramic (or other) bender 2 which converts the mechanical pulse to electric signals of the type represented by the traces 10A and 10B. The piesoceramic bender actually used is a Gulton, Piezoceramic bender element (R 205S) marketed by Piezo Products Division, Gulton Industries, Inc., 212 Durham Avenue, Metuchen, New Jersey 08840, U.S.A. which is a sandwich structure (see Fig. 4) which measures 2 inches in length, 0.5 inches in width and 0.02 inches in thickness.

The disposable retriever 3, as above noted, is held within the Teflon sleeve 5, the sleeve being attached by epoxy cement to the bender 2 which is secured to a base 6 in Fig. 1 and thence to a micromanipulator shown diagrammatically at 7 in Fig. 3. The micromanipulator 7, a trade device, allows placement of the transducer assemblies 1A and 1B, onto the corneal surfaces of the eyes. The manipulator allows movement of the transducer assemblies in three directions (x, y and z) in Fig. 3.

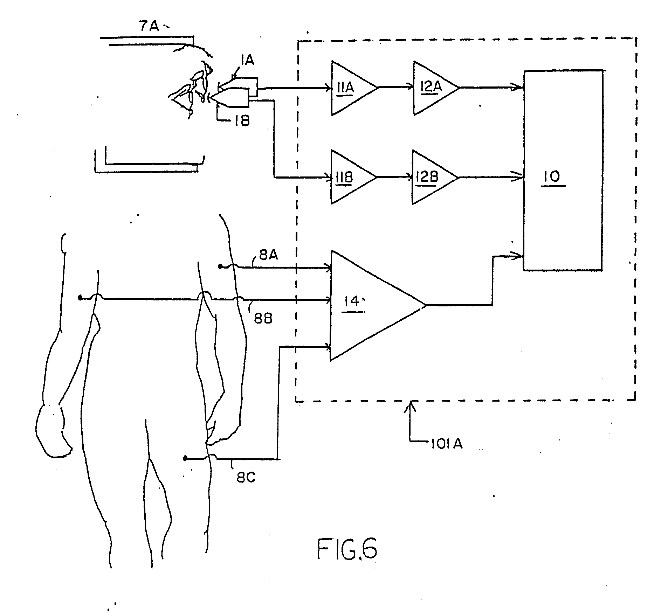

The procedure for obtaining the traces 10A, 10B and 10C is straightforward. For human OP recording, the patient is seated with the head immobilized on a headrest 7A (see Fig. 6) which is attached to the manipulator 7. The ECG leads 8A, 8B and 8C in Figs. 5 and 6 are placed in the known way. The patient holds both eyelids open while each lens retriever 3 is placed in contact with an eye. The transducers are left in contact with each eye for about three seconds and then retrieved; and ECG of the patient is taken at the same time. The session is over. The retrievers are removed and replaced by new sterilized elements. Synthetic tears may be used to overcome any dryness at the corneal surface; a topical corneal anesthetic may be used.

Returning to Fig. 5, the electrical portions of the system 101 includes coaxial cables 9A and 9B connected as inputs to the first and second isolation amplifier 11A and 11B to receive respectively a first signal from the first transducer and a second signal from the second transducer. Each of the amplifiers produces variable gain upon the input signal but each serves as well to insulate the patient against leakage currents and amplifier fault currents, a function which is complemented by the Teflon sleeve 5 and the plastic retriever 3. First and second variable filters 12A and 12B receive the amplified signals and supply outputs to provide the traces 10A and 10B. A voltage level detection circuit 13 serves to indicate to the operator when the transducer is in contact with eye and allows calibration of the system when needed.

The ECG input signals are amplified at 14 (another isolation amplifier), and filtered. A modular power supply 15 furnishes electric power to the various electrical elements just discussed.

The system 101A in Fig. 6 is similar to the system 101. The further figure is used mostly to show a patient in place on the headrest 7A.

The system 101 and 101A provide reliable and accurate mechanisms for recording mass screening to determine occlusions of extracranial arteries, but either can be used, with slight modification, to sense glaucoma. Glaucoma is one of the most common causes of blindness. It is a disease of the eye in which the intraocular pressure becomes pathologically high sometimes rising to as high as 70 millimeters of mercury. The average normal intraocular pressure is approximately 16 millimeters of mercury, with a range from 12 to 20. By determining the intraocular pressure, glaucoma can be diagnosed. The new measurement system disclosed herein with slight modification will measure intraocular pressure. It is the beauty of the piezoceramic transducer in that it bends/displaces to force. Therefore, when the transducer is in contact with the eye, a known small amount of displacement is applied to the transducer via the micromanipulator, causing the central portion of the cornea to be displaced inwardly. The amount of displacement of the cornea is directly related to the pressure inside the eye into the cornea, i.e. the intraocular pressure. The displacement of the piezoelectric crystal or the

- 10 -

intraocular pressure is given by the crystal as a voltage. With high intraocular pressure, i.e. glaucoma, the cornea will not be displaced inwardly as much as compred to the displacement with normal intraocular pressure; thus when a patient tested suffers from glaucoma a lower voltage will appear at the crystal output as compared to a nonglaucomic state. The method is noninvasive and the likelihood of infection and transmitted disease is greatly reduced.

1. Apparatus for noninvasively sensing ocular pulses of a mammal, which pulses serve as a basis for indicating a condition of the mammal which causes alterations in the arterial pulses, characterized by: piezoelectric transducer means to receive arterial pulses from the mammal and operable to convert the arterial pulses to a signal in the form of similar electric signals; and means connected to receive said signal and operable to permit deriviation of the information from which the condition of the mammal can be inferred.

2. Apparatus as defined in claim 1, characterized in that the disease or malfunctioning body part is a condition of glaucoma of said at least one eye, in which said means connected comprises display means operable to display said signals as a series of waveshapes, and in which said information is found in the shape of each waveshape, in said series of waveshapes.

3. Apparatus to achieve noninvasive measurement of ocular pulses in mammals, which pulses serve as a basis for determination of any occlusion of extracranial arteries of the mammal, characterized by:

(a) a first piezoelectric transducer means (1A) to receive ocular pulses from one eye of the mammal and operable to convert the ocular pulses to a first signal of similar electric pulses,

(b) second piezoelectric transducer means (1B) to receive ocular pulses from the other eye of the mammal and operable to convert the ocular pulses to a second signal of similar electric pulses,

(c) ECG transducer means (14) to record heartbeats of the mammal in the form of electric signals, and

(d) display means (10) connected to receive the first signal of similar electric pulses, the second signal of similar electric pulses, and the electric signal, and operable to display the same as a series of waveshapes to permit simultaneous observation and simultaneous comparison of the amplitudes and areas of a waveshape derived from each eye, the amount of distortion of the waveshape derived from each eye, and the delay of the waveshape from each eye with respect to the corresponding ECG signal, to permit an inference of any occlusion of the extracranial arteries of the mammal being checked therefor.

4. Apparatus as claimed in claim 3, wherein each piezoelectric transducer is characterized by:

a. eye contact means fitted to the surface of the eye, and which mechanically pulses in accordance with the ocular pulses of the eye,

b. a piezoceramic bender for converting the mechanical pulses of the eye contact means into a signal of similar electric pulses, and

c. means for attaching the eye contact means to the piezoelectric bender, so that the eye contact means may be connected to the piezoceramic bender easily and rapidly.

5. Apparatus according to claim 4, characterized by the eye contact means having an aperture, the aperture extending from the surface of the eye contact means making contact with the eye to at least one other surface of the eye contact means, to allow easy, rapid, and safe removal of the eye contact means from the surface of the eye.

6. Apparatus according to claim 3, characterized in that the display means comprises:

(a)  a first isolation amplifier which produces a first variable gain for the first signal of similar electric pulses received from the first piezoelectric transducer means, and which serves to insulate the mammal wearing the eye contact means from electric shock hazards by leakage currents, amplifier fault currents, and the like,

(b)  a second isolation amplifier which produces a second variable gain for the second signal of similar electric pulses received from the second piezoelectric transducer means, and which serves to insulate the mammal wearing the eye contact means from electric shock hazards such as leakage currents and amplifier fault currents, and the like,

(c)  a first variable filter which provides a first waveshaped signal for the first variable gain received from the first isolation amplifier,

(d)  a second variable filter which provides a second waveshaped signal for the second variable gain received from the second isolation amplifier, and

(e)  a readout device which receives the first waveshaped signal from the first variable filter, the second waveshaped signal from the second variable filter, and the electric signal from the ECG transducer, and operable to display the same as a series of waveshapes to permit simultaneous observations and simultaneous comparisons of the amplitudes and the areas of waveshapes derived from each eye, the amount of distortion of the waveshape derived from each eye, and the delay of the waveshape of the ECG signal, to permit an inference of any stenosis or occlusion of the extracranial arteries of the mammal being checked therefor.

7.  Apparatus as claimed in claim 4, characterized by:

(d)    a first three-dimensional micromanipulator upon which the first piezoelectric transducer is mounted, to allow proper positioning of the first eye contact means to the surface of the one eye, and to insure the first piezoelectric transducer remains stationary while measurements of the ocular pulse of the one eye are taken, and

(e)    a second three-dimensional micromanipulator upon which the second piezoelectric transducer is mounted, to allow proper positioning of the second eye contact means to surface of the other eye, and to insure the second piezoelectric transducer remains stationary while measurements of the ocular pulse of the other eye are taken.

8.    A noninvasive method of measuring the ocular pulses in mammals, which pulses serve as a basis for determination of any stenosis or occlusion in extracranial arteries of the mammal, characterized by:

(a)    immobilizing the head of the mammal,

(b)    attaching an ECG lead to at least three limbs of the mammal, to record heartbeats of the mammal in the form of electric signals,

(c)    fitting a first piezoelectric transducer onto the surface of one eye of the mammal to convert the ocular pulse received by the first piezoelectric transducer to a first signal of similar electric pulses,

(d)    fitting a second piezoelectric transducer onto the surface of the other eye of the mammal to convert the ocular pulse received by the second piezoelectric transducer to a second signal of similar electric pulses,

(e)    displaying waveshapes of the electric signal from the ECG transducer, the first signal of similar electric pulses from the first piezoelectric transducer, and the second signal of similar electric pulses from the second piezoelectric transducer, and

(f) comparing the series of waveshapes to permit simultaneous observation and simultaneous comparison of characteristics of the waveshapes derived from each eye and the ECG to permit an inference of any stenosis or occlusion of the extracranial arteries of the mammal being examined therefor;

9. A method as claimed in claim 8, characterized in that the ECG leads are attached to three limbs of the mammal.

10. A method as claimed in claim 8, characterized in that said characteristics include amplitudes and areas of the waveshapes derived from each eye, the amounts of distortion of the waveshapes derived from each eye, and the delay time of the waveshapes from each eye with respect to the corresponding ECG signal, wherein said amplitudes, areas, distortion and delay time serve to permit said inference.

0114499

FIG.2

FIG.1

FIG.3

FIG.4

FIG.5

0114499

FIG.6

7A

IA
IB

11A 12A

11B 12B

10

8A
8B
14

8C

101A

3/3

0114499